# EUROPÄISCHE PATENTSCHRIFT

(11) **EP 1 262 751 B1**
(45) Veröffentlichungstag und Bekanntmachung des Hinweises auf die Patenterteilung: **06.04.2011**
(21) Anmeldenummer: 02011262.9
(22) Anmeldetag: 22.05.2002
(51) Int. Cl.: G01J 3/10, G01J 3/50, G01N 21/17

(54) **Vorrichtung und Verfahren zur Lichtanalyse**
Apparatus and method for analysing light
Dispositif et procédé d'analyse de lumière

(30) Priorität: 01.06.2001 DE 10126887; 20.07.2001 US 307001 P; 09.10.2001 US 327906 P
(43) Veröffentlichungstag der Anmeldung: 04.12.2002
(73) Patentinhaber: Ivoclar Vivadent AG, 9494 Schaan (LI)
(72) Erfinder: Kerschbaumer, Harald, 6833 Klaus (AT); Pokorny, Walter, 6712 Thüringen (AT); Rohner, Gottfried, 9450 Altstätten (CH); Pye, Graham, 3600 Genk (BE)
(74) Vertreter: Baronetzky, Klaus

(56) Entgegenhaltungen:
- WO-A-00/69333
- US-A- 5 038 258
- US-A- 5 541 419
- US-A- 5 910 841
- US-A- 5 990 468
- US-A- 6 032 071
- PATENT ABSTRACTS OF JAPAN Bd. 0182, Nr. 47 (P-1735), 11. Mai 1994 (1994-05-11) & JP 6 028368 A (IIZERU SHARP SEMICONDUCTOR:KK; others: 02), 4. Februar 1994 (1994-02-04)

## Beschreibung

Die vorliegende Erfindung betrifft eine Lichtanalysevorrichtung, gemäß dem Oberbegriff von Anspruch 1 und eine Dentalkamera; gemäß dem Oberbegriff von Anspruch 11 sowie ein Verfahren für das Abbilden von Zahnoberflächen gemäß dem Oberbegriff von Anspruch 14.

Eine derartige Lichtanalyse- oder Farbfestlegungsvorrichtung ist seit Längerem bekannt, beispielsweise aus der DE-OS 195 34 517. Über drei Lichtquellen wird bei dieser Lösung eine Zahnoberfläche beaufschlagt und über drei entsprechende Sensoren werden die je gemessenen Helligkeitswerte für die drei Grundfarben erfasst. Die Auswertung soll hierbei eine möglichst geringe Farbabweichung zu den zur Verfügung stehenden Zahnfarben ergeben.

Weitere Lichtanalysevorrichtungen sind aus US 6032071, US 5038258 und US 5910841 bekannt.

Ferner ist es seit Längerem bekannt, dass Reflexionen das Erfassungs- und Auswertergebnis verfälschen. Durch die Anordnung von Lichtquellen in unterschiedlichen Winkeln mit bei unterschiedlichen Spektren lässt sich die Neigung zu Reflexionen etwas kompensieren.

Es ist seit langem bekannt, dass aufgrund der geringen Rautiefe der Zahnoberflächen Reflexionen das Ergebnis der Auswertung verfälschen. Um möglichst geringe Reflexionen zu erhalten, ist es bekannt, Titanoxidpuder als Kontrastpuder auf die Zahnoberfläche aufzubringen. Je nach Schichtstärke ist jedoch eine Verfälschung der Farbwerte nicht auszuschließen.

Um die Nachteile von Kontrastpuder zu vermeiden, ist es bereits vorgeschlagen worden (vgl. die DE-OS 40 37 007) eine Fluoreszenzfarbe zu verwenden, die Grün absorbiert und Gelb oder Rot emittiert. Ein deutlicher Nachteil einer derartigen Fluoreszenzfarbe liegt jedoch darin begründet, dass die Farbe verfälscht wird, so dass eine Kompensation erforderlich ist, wenn die Verschiebung erfasst werden soll. Auch ist es schwierig, die Fluoreszenzfarbe exakt in gleichmäßiger Stärke aufzubringen, so dass sich diese Lösung nicht durchgesetzt hat.

Für die Beurteilung der Oberflächenstruktur ist es im Gegensatz zur Farbbeurteilung wünschenswert, Schatten zu Erfassen, so dass die Struktur erkennbar ist. Auch hierbei stört die Aufbringung von Kontrastpuder oder fluoreszierender Farbe, denn derartige Abschattungen und dergleichen werden durch derartige Mittel reduziert oder aufgehoben.

Ferner ist es derzeit vorgeschlagen worden, ein drehbares Polarisationsfilter zu verwenden, das je nach Wahl des Anwenders die Reflexionen bei polarisiertem Licht zuläßt oder stufenlos vermindert. Diese Lösung erlaubt zwar den Vergleich einer Zahnoberfläche mit und ohne Reflexionen. Das Auswertungsergebnis hängt jedoch sehr stark davon ab, aus welchem Winkel die Lichtquelle die Zahnoberfläche beaufschlagt. Bei seitlicher Beaufschlagung ist häufig der von der Lichtquelle entfernte Bereich der ZahnOberfläche zu stark abgedunkelt und die dortige Struktur ist gebenenfalls nicht mehr erkennbar.

Daher liegt der Erfindung die Aufgabe zugrunde, eine Lichtanalysevorrichtung gemäß dem Oberbegriff von Anspruch 1 und eine Dentalkamera gemäß dem Oberbegriff von Anspruch 11 zu schaffen, die sowohl die Farbe als auch die Struktur einer Zahnoberfläche verbessert zu erfassen vermag.

In einer bevorzugten Ausführungsform der Erfindung ist eine Lichtanalysekamera mit einem Gehäuse vorgesehen, das mit einer Hand handhabbar ist. Die Kamera weist eine erste und eine zweite Lichtquelle auf, die Licht auf eine Zahnoberfläche werfen. Die erste Lichtquelle erzeugt bevorzugt ein im Wesentlichen nicht polarisiertes Licht. Die zweite Lichtquelle erzeugt ein zweites Licht, das entlang einer ersten Achse polarisiert ist, bevorzugt durch ein Polarisationsfilter. Ein Lichtsammelelement sammelt das erste und zweite von der Zahnoberfläche reflektierte Licht das erste und zweite von der Zahnoberfläche reflektierte Licht und weist eine Aufnahmevorrichtung auf, die ein dem reflektierten Licht entsprechendes Bild erfasst und erzeugt. Das Lichtsammelelement weist ein Polarisationsfilter auf, das das reflektierte zweite Licht entlang einer zweiten Achse in einem Winkel zu der ersten Achse reflektiert, um Blenden und Reflexionen zu verhindern. Bevorzugt wird ein Bild von dem Licht aus jeder der Lichtquellen erzeugt, und ein mit den Lichtquellen verbundener Zeitgeber und ein Sammelelement aktiviert die erste und die zweite Lichtquelle nacheinander, so dass ein erstes und ein zweites Bild nacheinander erzeugt werden.

Die bevorzugte Kamera enthält einen Lichtkanal, der Licht durchlässt und radial aus anderen Quellen in den Kanal eintretendes Licht blockiert. Die erste und die zweite Lichtquelle sind in dieser Ausführungsform dem Lichtsammelelement benachbart entlang einer Richtung angeordnet, die zum Lichtkanal hin abgewinkelt ist und bevorzugt nach vorne zur Position der Zahnoberfläche hin geneigt ist. Zudem sind der erste und der zweite Pfad in ungleichen Winkeln zu der Position der Zahnoberfläche angeordnet.

In dem Verfahren des erfindungsgemäßen Abbildens einer Zahnoberfläche wird die Zahnoberfläche mit einem ersten Licht von einer ersten Quelle beaufschlagt, beispielweise einer Kamera. Das erste von der Zahnoberfläche reflektierte Licht wird in der Quelle bzw. Kamera gesammelt und es wird ein erstes Bild erzeugt, das dem ersten gesammelten Licht entspricht. Die Zahnoberfläche wird mit einem zweiten Licht aus der Quelle bzw. Kamera beaufschlagt, wobei das zweite Licht entlang einer ersten Achse polarisiert wird. Das zweite von der Zahnoberfläche reflektierte Licht wird.zum Polarisieren des zweiten Lichts entlang einer zweiten durch ein Polarisationsfilter geleitet und gesammelt, so dass ein zweites dem zweiten gesammelten Licht entsprechendes Bild entsteht. Während das Bild des ersten Lichts den Aufbau des Zahnes detailliert dank des Blendens und der Reflexionen im Bild darzustellen vermag, ist das Bild des zweiten Lichts frei, oder freier, von unerwünschten Reflexionen, was eine genauere Farbanalyse erlaubt. Eines der Lichter wird vor dem anderen beaufschlagt und gesammelt, und die Beaufschlagung wird bevorzugt in einem Abstand von etwa 2 Sekunden vorgenommen. Das bevorzugte Verfahren umfasst auch das Blockieren von Licht aus anderen Quellen als der Quelle bzw. Kamera. Die Bilder werden bevorzugt elektronisch erzeugt. Wenn die Lichtquelle eine Kamera ist, können die Bilder in der Kamera angesehen werden oder elektronisch an einen anderen Ort übertragen werden, wo sie dann angesehen werden können.

Indem zwei Lichtquellen verwendet werden, die die Zahnoberfläche zu verschiedenen Zeitpunkten und aus unterschiedlichen Winkeln beaufschlagen, werden Abschattungen, die bei der Aufnahme des Bildes der Zahnoberfläche bei Belichtung durch eine Lichtquelle entstehen, kompensiert, wobei es sich erfindungsgemäß überraschend erwies, dass die Filterung des Lichts durch lediglich einen Polarisationsfilter die Beurteilung der Abschattierungen nicht erschwert, die Informationen über die Beschaffenheit des Zahnes liefern, wenn die treffende Lichtquelle polarisationsfilterfrei die Zahnoberfläche bestrahlt. Erfindungsgemäß ist es insofern besonders günstig, dass durch die Beaufschlagung der Zahnoberfläche mit einer Lichtquelle mit Polarisationsfilter ein reflexionsfreies Bild der Zahnoberfläche aufgenommen werden kann, während durch die Beaufschlagung mit der anderen Lichtquelle, die keinen Polarisationsfilter aufweist und die gegenüber der einen Lichtquelle in einem anderen Winkel angeordnet ist, die Erfassung von Strukturen gut möglich ist.

Bevorzugt sind die Lichtquellen als Weißlichtquellen ausgebildet. Ein Referenzmuster wird mit den gleichen Lichtquellen beaufschlagt, um Reflexionen zu schaffen, die mit der gleichen Aufnahmevorrichtung eines Lichtsammelelements für eine elektronische Kompensation der Zähne aufgenommen werden. Das Referenzmuster ist bevorzugt knapp unterhalb oder überhalb des Zahns/der Zähne mit der aufgenommenen Zahnoberfläche angeordnet.

Erfindungsgemäß ist es ferner vorgesehen, dass mit zwei Lichtquellen die Lichtbeaufschlagung der Zahnoberfläche von zwei unterschiedlichen Seiten erfolgt, was die Ausleuchtung zur Erfassung der Farbe und Struktur verbessert. Dadurch, dass die Lichtquellen abwechselnd eingeschaltet werden, lässt sich die Struktur des Zahns recht deutlich erkennen und bei dem herzustellenden Zahnmodell in entsprechender Weise nachmodellieren.

Erfindungsgemäß ist es ferner vorgesehen, dass durch die gemeinsame Aufnahme von Bildern, die durch unterschiedliche Lichtquellen hervorgerufen werden, zugleich auch eine Überprüfung der Lichtquellen und deren Farbspektren wirksam möglich ist. Hierzu wird eine sogenannte Weißkarte anstelle der Zahnoberfläche eingesetzt und die gemessenen Farbwerte jeder Lichtquelle werden für den Weißabgleich verwendet. Darüber hinaus ist im Strahlengang zwischen der Zahnoberfläche und der Aufnahmevorrichtung eine optische Linse vorgesehen. Mit einer derartigen optischen Linse ist es möglich, das Bild der Zahnoberfläche auf die Aufnahmevorrichtung zu fokussieren, um dort eine möglichst präzise Wiedergabe der tatsächlichen Zahnoberfläche aufnehmen zu können.

Zudem ist es vorgesehen, zusätzlich zu den Lichtquellen, die in verschiedenen Winkeln zu der Zahnober fläche angeordnet sind und diese schräg bestrahlen, weitere Lichtquellen vorzusehen, die je auf der anderen Seite der Zahnoberfläche angebracht sind. Beispielsweise ist es möglich, eine Lichtquelle mit Polarisationsfilter im Winkel von etwa 45° zur Zahnoberfläche anzuordnen und die Zahnoberfläche aus diesem Winkel bestrahlen zu lassen. Eine weitere Lichtquelle ohne Polarisationsfilter kann im Winkel von 70° zur Zahnober fläche angeordnet sein, so dass Abschattierungen besonders deutlich werden. Durch die Anordnung der Lichtquellen lässt sich die erwünschte Erfassung sowohl der Farbe ohne Reflexionen als auch der Struktur optimal bereitstellen.

Weitere Vorteile, Einzelheiten und Merkmale ergeben sich aus der nachfolgenden Beschreibung eines Ausführungsbeispiels anhand der Zeichnung.

### Es zeigen:

- Fig. 1: eine schematische Ansicht einer erfindungsgemäßen Farbfestlegungsvorrichtung in einer Ausführungsform;
- Fig. 2: eine Abbildung von Aufnahmen der Aufnahmevorrichtung, wobei links eine Aufnahme über die Lichtquelle ohne Polarisationsfilter und rechts eine Aufnahme über die Lichtquelle mit Polarisationsfilter dargestellt ist;
- Fig. 3: eine Seitenansicht einer Ausführungsform der erfindungsgemäßen Dentalkamera;
- Fig.: 4 eine Seitenansicht der optischen Bauteils der Kamera;
- Fig. 5: eine Seitenansicht des optischen Bauteils einer anderen Ausführungsform mit einer Zoomfunktion; und
- Fig. 6: bis 8 Ansichten der Rückseite der erfindungsgemäßen Kamera, die die durch Verwenden der Zoomfunktion entstandenen Bilder erzeugt.

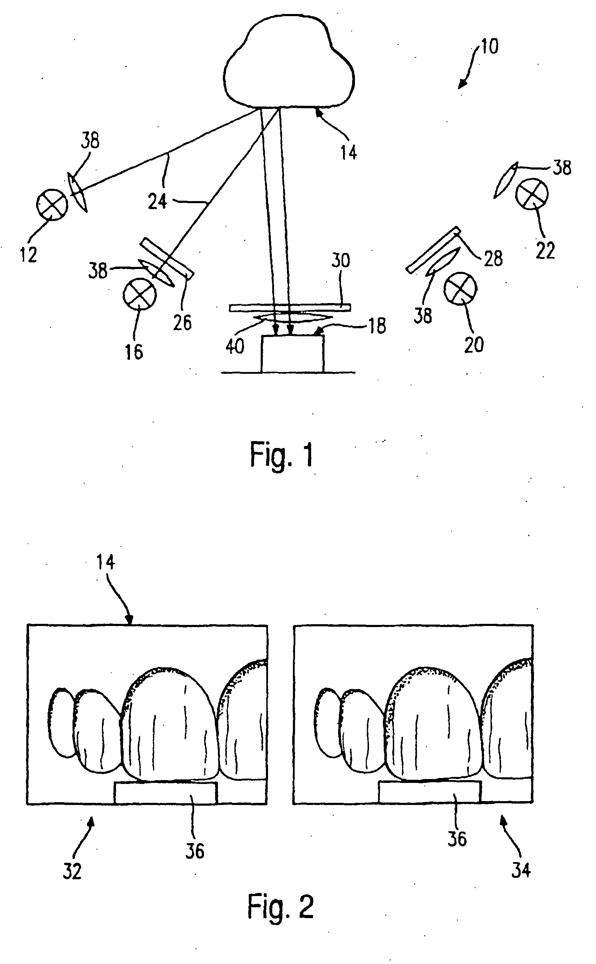

Die Farbfestlegungsvorrichtung 10 gemäß Fig. 1 weist eine Lichtquelle 12 auf, die recht weit seitlich vor einer Zahnoberfläche 14 angeordnet ist. Ferner ist eine Lichtquelle 16 vorgesehen, die auf der gleichen Seite wie die Lichtquelle 12 schräg vor der Zahnoberfläche 14 angeordnet ist.

Zentral und mittig vor der Zahnoberfläche 14 ist eine Aufnahmevorrichtung 18 angeordnet, die das von der Zahnoberfläche 14 reflektierte Licht erfassen soll.

Symmetrisch zu der Lichtquelle 16 ist schräg vor der Zahnoberfläche 14, jedoch auf der anderen Seite, eine Lichtquelle 20 angeordnet. Ferner ist bei der in Fig. 1 dargestellten Ausführungsform eine vierte Lichtquelle 22 recht weit seitlich vor der Zahnoberfläche 14 angeordnet. Die Lichtquelle 22 ist symmetrisch zu der Lichtquelle 12 angeordnet. Bevorzugt sind die Lichtquellen 16 bis 20 im Wesentlichen im selben Winkel von der Aufnahemvorrichtung 18 angeordnet. Die Lichtquellen 12 bis 22 sind entsprechend im Wesentlichen im selben Winkel von der Aufnahmevorrichtung 18 angeordnet, der aber größer ist als der Winkel, in dem die Lichtquellen 16 bis 22 angeordnet sind.

Jede Lichtquelle 12 bis 22 besteht aus einer Mehrzahl von LEDs, so dass eine entsprechend hohe Lichtleistung bereitgestellt wird. Alle Lichtquellen 12 bis 22 sind als weiße Leuchtdioden ausgebildet. Sie emittieren Licht 24, wie es beispielsweise für die Lichtquellen 12 und 16 dargestellt ist, das auf die Zahnober fläche 14 fällt und dort reflektiert wird und zur Aufnahme vorrichtung 18 gelangt.

Erfindungsgemäß ist nun die Lichtquelle 12 und die Lichtquelle 22 so ausgebildet, dass sie die Zahnoberfläche 14 direkt beauf-' schlagt, also ohne einen Filter. Demgegenüber ist vor der Lichtquelle 16 und auch vor der Lichtquelle 20 je ein Polarisationsfilter 26 und 28 vorgesehen, die von dem Licht 24 durchtreten werden, bevor es auf die Zahnoberfläche, 14 gelangt. Beispielsweise sind die Polarisationsfilter 26 und 28 so angebracht, dass sie das Licht vertikal polarisieren.

Ferner ist im Strahlengang zwischen der Zahnoberfläche 14 und der Aufnahmevorrichtung 18 ein weiterer Polarisationsfilter 30 angebracht. Die Polarisationsrichtung des Polarisationsfilters 30 ist demgegenüber horizontal. Mit dieser Anordnung lassen sich in an sich bekannter Weise Reflexionen der Zahnoberfläche dann unterdrücken, wenn die Lichtquellen 16 und 20 eingeschaltet sind.

Die Lichtquellen 12 bis 22 sind bevorzugt von Reflektoren zur Erhöhung der Lichtausbeute umgeben, und das von den den Lichtquellen emittierte Licht wird in je Sammellinsen 38 gesammelt. Bevorzugt sind diese Sammellinsen 38 so ausgebildet, dass sie farbtreu sind und gleich keine Verfälschung des emittierten Farbspektrums erzeugen.

In entsprechender Weise ist zwischen der Zahnoberfläche 14 und der Aufnahmevorrichtung 18 eine Sammellinse 40 vorgesehen, die das Bild der Zahnoberfläche 14 auf die Aufnahmevorrichtung 18 fokussiert. Die Aufnahmevorrichtung 18 weist einen CCD-Sensor oder einen beliebigen anderen geeigneten Sensor auf.

Aus Fig. 2 ist ersichtlich, in welcher Weise das Bild der Zahnoberfläche 14 erfasst werden kann. Bevorzugt wird mindestens ein ganzer Zahn abgebildet, obwohl mindestens zwei Zähne in der bevorzugten Ausführungsform angesehen werden können. In den Figuren sind zwischen drei und vier Zähnen in jedem Bild enthalten. Das in Fig. 2 links dargestellte Bild 32 wird erzeugt, indem die Lichtquelle 12 eingeschaltet wird. Die Reflexionen, die durch das Beaufschlagen der Zahnoberfläche von der Lichtquelle 12 entstehen, lassen die Struktur der Zahnoberfläche erkennen. Demgegenüber wird das in Fig. 2 rechts dargestellte Bild 34 erzeugt, in dem die Lichtquelle 16 eingeschaltet wird. Durch die Polarisation des einfallenden Lichtes in einer zur Polarisationsrichtung des reflektierten Lichts senkrechten Richtung werden die Relexionen ausgeschaltet und eine Farbbestimmung ist leichter möglich.

Die Bilder 32 und 34 zeigen zusätzlich zu den Zahnoberflächen 14 je Referenzmuster 36, die unmittelbar benachbart und unter halb der Zahnoberfläche eingeblendet sind. Bevorzugt sind entsprechende Referenzmusterkärtchen so angeordnet, dass sie unmittelbar benachbart den Zähnen aufgenommen werden können.

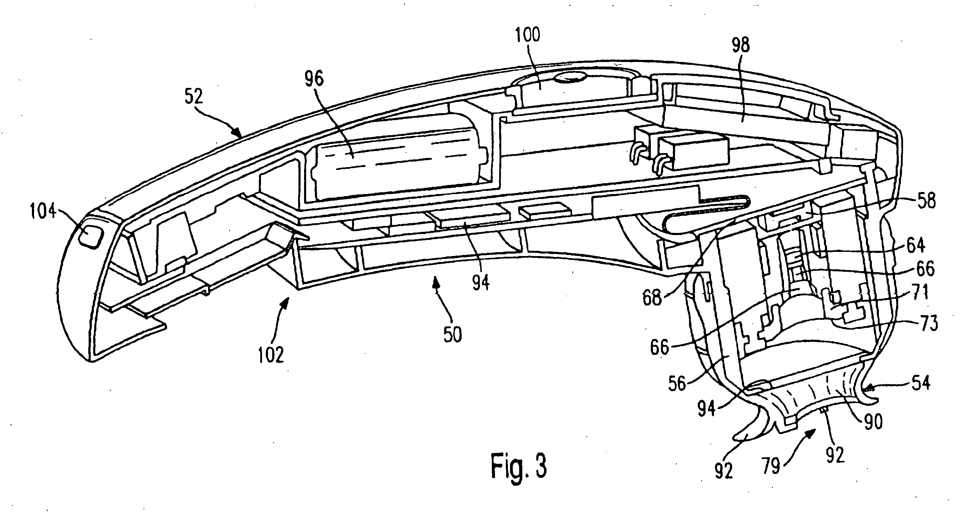

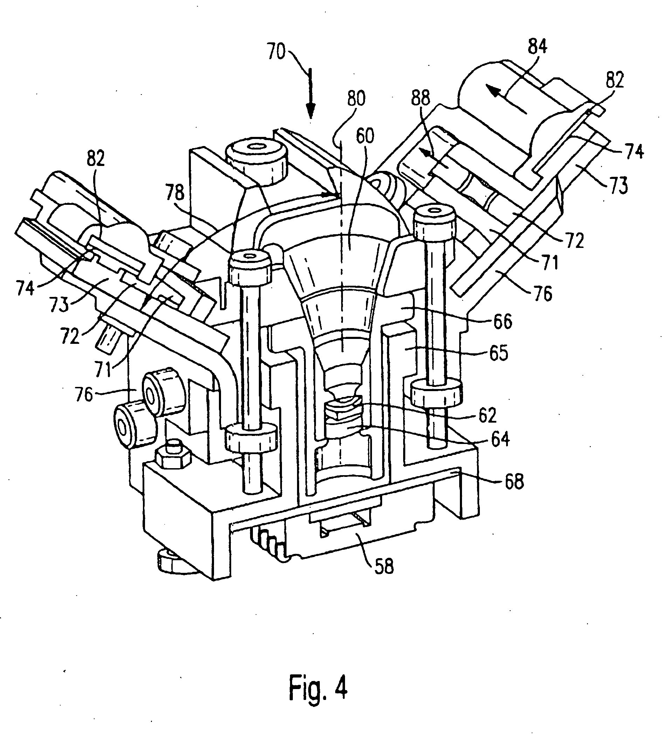

Figuren 3 und 4 zeigen eine bevorzugte Ausführungsform einer Kamera 50 einschließlich der Farbfestlegungsvorrichtung mit einem Gehäuse 52 und einem Mundstück 54, das an einer Kupplung 56 des Gehäuses 52 befestigt ist. Eine Aufnahmevorrichtung 58, die vorzugsweise ein Lichtsammelelement, wie beispielsweise einen CCD-Chip, aufweist, ist vorzugsweise im Wesentlichen koaxial zu einem Lichtsammelkanal 60 angeordnet, der vorzugsweise verhindert, das Licht dirket von den Lichtquellen in der Kamera eindringt.

In dem Lichtsammelkanal 60 sind vorzugsweise ein Infrarotfilter 62 und eine oder mehrere Kameralinsen 64 angeordnet, die auf einem beweglichen Fokussierteil 66 montiert sind. Das Fökussierteil 64 wird vorzugsweise von einer Elektronik und einem Motor in der Kamera 50 gesteuert, so dass sich das Fokussierteil 64 in die Richtungen 65 bewegt, um so den Abstand zwischen der Linse 64 und der Aufnahmevorrichtung 58 zu variieren, damit das gesammelte reflektierte Licht angemessen auf dem CCD fokussiert werden kann und ein klares Bild entsteht. Ein Polarisationsfilter 68 ist entlang dem reflektierten Pfad 70 vor der Aufnahmevorrichtung 58 angeordnet.

Die Lichtquellen 72 und 74 sind vorzugsweise jeweils auf der Seite des Lichtsammelkanals 60 angeordnet. Die bevorzugten Lichtquellen 71 und 73 weisen Reihen von LEDs auf, die ein weißes Licht erzeugen. In der dargestellten Ausführungsform sind die LEDs 72 und 74 auf Stützen 76 montiert, die sich entlang einer zum Lichtkanal hin abgewinkelten Richtung erstrecken, vorzugsweise nach vorne hin zur Objektposition 79, in der sich die zu erfassenden Zähne befinden, von denen ein Bild erzeugt wird. Die Stützen 76 sind vorzugsweise in einem Winkel 78 zur Achse 80 des Lichtkanals von etwa zwischen 10° und 80° angeordnet, besser jedoch zwischen 30° und 60°, und am besten zwischen 40° und 50°. Die bevorzugten Stützen 76 sind gerade, obwohl Alternativen gekrümmt sein können, um ein optimales Plazieren der Lichtquellen zu erreichen. Die vorliegende Ausführungsform umfasst eine Stütze auf zwei Seiten des Lichtsammelkanals 20, aber es kann eine auch andere Anzahl von Stützen und Lichtguellenpositionen eingesetzt werden. Eine Ausführungsform weist beispielsweise Lichtquellen auf, die im Wesentlichen alle Seiten des Lichtsammelkanals 60 umgeben, und die Stützen können als eine Einheit ausgebildet sein.

Die Position und Anordnung der Lichtquellen 72 und 74 wird vorzugsweise so gewählt, dass die Lichtpfade die Objektposition 79 aus verschiedenen Winkeln erreichen. Mit dieser Anordnung kann eine die Effektivität der Lichtquellen bei der Ausleuchtung der Zähne zur Farbanalyse oder zur Visualisierung der Zähne maximiert werden.

Die Lichtquellen 71 erzeugen vorzugsweise im Wesentlichen nicht polarisiertes Licht ohne Polarisationsfilter zwischen der Objektposition 79 und der LEDs 72 entlang des Lichtpfads 88. Wahlweise kann ein Polarisationsfilter mit den Lichtquellen 71 eingesetzt werden, dessen Orientierung von der Polarisationsachse des Polarisationsfilters 68 kaum oder nur gering abweicht. Zusätzlich schließt die in der Ausführungsform gemäß Figuren 3 und 4 gezeigte Lichtquelle 72 keine Linse vor den LEDs 72 ein, alternative Ausführungsformen schließen aber Linsen im von diesen LEDs kommenden Lichtpfad ein.

Gemäß Fig. 3 ist die Achse des optischen Bauteils der Kamera 50 im Wesentlichen koaxial mit dem Mundstück 54 und der Kupplung 56 ausgerichtet, und der Hauptlichtkanal 90 erstreckt sich durch die Kupplung 56 und das Mundstück 54, um das Licht aus den Lichtquellen 72 und 74 an den Zahn in der Objektposition 79 und das vom Zahn reflektierte Licht zurück in den Lichtsammelkanal 60 gelangen zu lassen.

Das Mundstück 54 der gezeigten Ausführungsform setzt den Abstand zwischen der Kamera und den Zähnen fest und enthält sich radial erstreckende Flügel 92, die zwischen die Lippen des Patienten und die Zähne bzw. den Gaumen geführt werden, um zu verhindern, dass Licht aus anderen Quellen in die Kamera eindringt und die zu erzeugenden Bilder beeinflusst. Bissvorsprünge 92, auf die der Patient aufbeissen soll, um die Zähne in Position zur Kamera zu halten, erstrecken sich vorzugsweise von den Flügeln nach vorne. Das Kameragehäuse 52 enthält vorzugsweise ein transparentes Schutzfenster, das vorzugsweise im Kuppelabschnitt 56 angeordnet ist, um zu verhindern, dass Materie aus dem Mund des Patienten in die Kamera gelangt. Ein Farb-Referenzmuster 93 ist im Mundstück 54 angeordnet, das in den erzeugten Bildern von den Zähnen erscheinen soll, da das Muster 93 eine bestimmte Farbe aufweist, vorzugsweise weiß, und so wird ein Vergleich der Zahnfarbe möglich.

In dem bevorzugten Gehäuse 52 befindet sich auch die Elektronik 94 zur Steuerung der Kamerafunktionen, Erzeugung der Bilder von den Signalen des CCDs und zum Speichern und Übertragen der Daten, einschließlich Bilddateien, an einen anderen Computer oder ein anderes elektronisches Gerät. Die Elektronik umfasst ein Speichermedium, wie beispielsweise ein digitales Speichermedium zum Speichern der Bilder und anderen Daten. Ebenfalls im Gehäuse untergebracht sind eine Energiequelle 96, wie beispielsweise eine Batterie oder ein Akkumulator zum Betreiben der Kamera, eine Anzeige 98, bevorzugt ein LCD-Farbbildschirm, die das gerade erzeugte Bild und die gespeicherten Bilder anzeigt, und ein Tastenfeld 100, das eine Schnittstelle für den Bediener zum Bedienen der Kamera liefert. Vorzugsweise werden mit der Kamerasteuerung verbundene Menüsymbole und Informationen zu den Bildern in der Anzeige 98 angezeigt. Eine transparente Schutzscheibe ist außerhalb der Anzeige 98 angeordnet, so dass die Anzeige vorzugsweise von einer Seite der Kamera 50 angesehen werden kann, die entgegengesetzt zu der Objektposition ist. Viele dieser Bauteile sind in einem länglichen Handstück 102 des Gehäuses angeordnet, das sich vorzugsweise in einem nach unten gerichteten Winkel vom Hauptlichtkanal 90 erstreckt. Vorzugsweise an den unteren Enden des Handstücks 102 ist eine Schnittstelle 104, wie beispielsweise eine USB-Schnittstelle, vorgesehen, die mit einem Computer oder anderen elektronischen Geräten verbunden werden kann und so Daten übertragen werden können.

Die Kamera 50 weist vorzugsweise einen Zeitgeber in der elektronischen Schaltkreisanordnung auf, der ein Bild aus dem Licht von der ersten und der zweiten Quelle erzeugen kann, vorzugsweise auf einen einzigen Knopfdruck auf einer der Tasten 100 durch den Bediener hin. Diese Bilder werden vorzugsweise mit etwa 2 Sekunden Abstand gemacht, bessern mit etwa 1 Sekunde Abstand, am besten mit etwa 1/2 Sekunde Abstand oder weniger.

Gemäß Figuren 3 oder 4 sind die Lichtquellen benachbart dem vorderen Ende der Lichtsammelvorrichtung und des Lichtsammelkanals 60 angeordnet und liefern so das Licht direkt an die Objektposition, wodurch keine Lichtleiter, wie beispielsweise faseroptische Leiter, Prismen oder Spiegel, mehr notwendig sind, um das Licht zur Objektposition 92 zu leiten. In einer alternativen Ausführungsform ist jedoch eine oder mehrere der Lichtquellen gut hinter dem vorderen Ende des Lichtsammelkanals angeordnet, und die Objektposition wird mit dem Licht aus den Lichtquellen beaufschlagt, indem das Licht durch optische Leiter geleitet wird oder durch Prismen und Spiegel reflektiert wird.

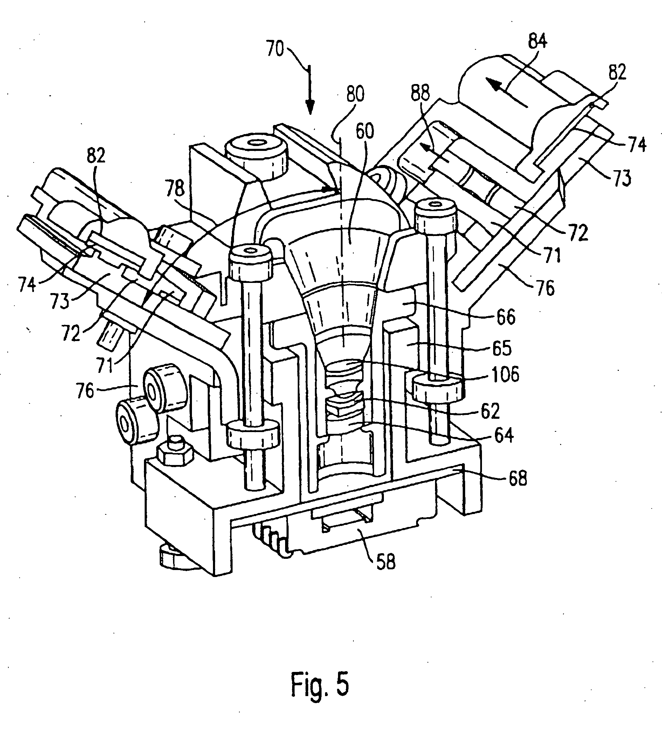

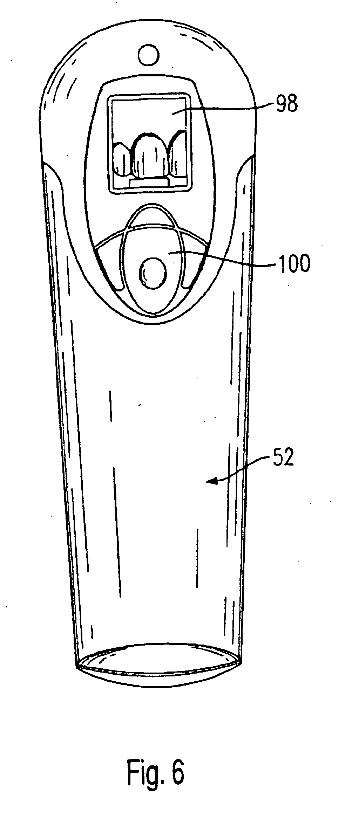

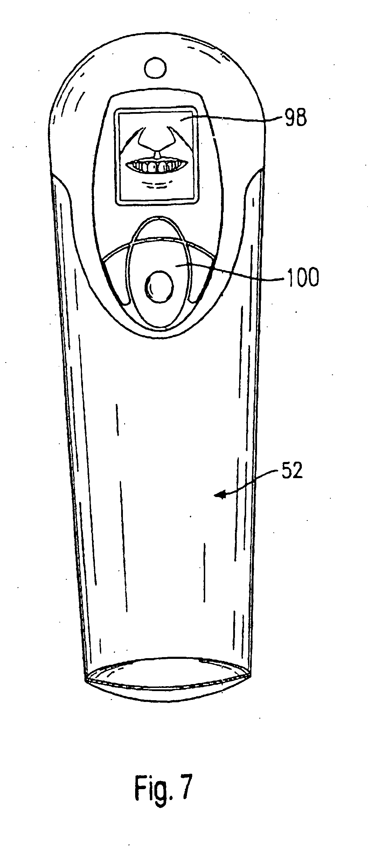

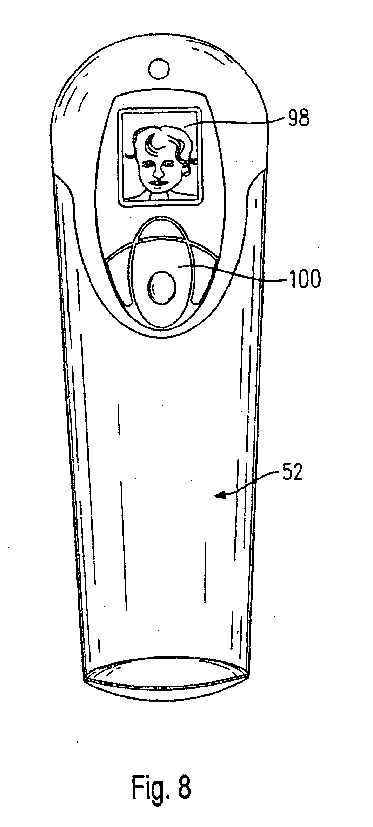

Die in Fig. 5 gezeigte Ausführungsform enthält ein optisches Zoombauteil 106 zum Variieren der Brennweite der Kamera. Das Zoombauteil 106 selbst weist eine oder mehrere Linsen auf und wird vorzugsweise von der Elektronik 94 und einem Motor gesteuert, um von einem Weitwinkelbild, siehe Fig. 6, in dem ein, zwei, drei oder vier Zähne beispielsweise im Bild zu sehen sind, zu einem Bild und einer Linsenanordnung zu gelangen, das/die zu einem Telefoto, einer Nahwinkel-Anordnung bzw. einem Nahwinkelbild neigt, in dem der ganze Mund, siehe Fig. 7, oder das Gesicht eines Patienten, siehe Fig. 8, zu sehen ist.

Während hier illustrative Ausführungsformen der Erfindung offenbart sind, versteht es sich, dass zahlreichen Modifikationen und andere Ausführungsformen von Fachleuten abgeleitet werden können. Beispielsweise blockiert oder reduziert eine Lichtquelle oder deren Filter in einer alternativen Ausführungsform Licht mit bestimmten Merkmalen des Lichts der anderen Lichtquelle, während in der bevorzugten Ausführungsform ein Polarisationsfilter in einer der Lichtquellen verwendet wird. Daher versteht es sich, dass die anhängigen Ansprüche all diese Modifikationen und Ausführungsformen abdecken soll, die im Sinne und im Schutzumfang der vorliegenden Erfindung liegen.

## Patentansprüche

1. Lichtanalysevorrichtung für den Dentalbereich, die Folgendes aufweist:
- eine erste Lichtquelle (12), die eine Zahnoberfläche (14) mit nicht polarisiertem Licht beaufschlagt, wobei ein erstes reflektiertes Licht erzeugt wird;
- eine zweite Lichtquelle (16), die die Zahnoberfläche (14) mit entlang einer ersten Achse polarisiertem Licht beaufschlagt, wobei ein zweites reflektiertes Licht erzeugt wird;
- ein Lichtsammelelement (18), das das erste und das zweite reflektierte Licht von der Zahnoberfläche (14) aufnimmt und eine Aufnahmevorrichtung aufweist, mit welcher ein dem reflektierten Licht entsprechendes Bild aufnehmbar und erzeugbar ist, wobei das Lichtsammelelement (18) ein Polarisationsfilter (30) aufweist, das so angeordnet ist,
dass es das zweite reflektierte Licht entlang einer zweiten Achse in einem Winkel zu der ersten Achse polarisiert,
**dadurch gekennzeichnet, dass** die Lichtquellen (12, 16) in verschiedenen Winkeln zu der Zahnoberfläche angeordnet sind und diese schräg bestrahlen und dass symmetrisch zu der ersten Lichtquelle (12) schräg vor der Zahnoberfläche (14), jedoch auf der anderen Seite der Zahnoberfläche (14) eine dritte Lichtquelle (22) angeordnet ist, wobei die erste und die dritte Lichtquelle (20) die Zahnoberfläche mit nicht polarisiertem Licht beaufschlagen, und wobei symmetrisch zu der zweiten Lichtquelle (16) schräg vor der Zahnoberfläche (14), jedoch auf der anderen Seite der Zahnoberfläche (14), eine vierte Lichtquelle (20) vorgesehen ist, wobei die zweite und die vierte Lichtquelle (16, 20) die Zahnoberfläche (14) mit polarisiertem Licht beaufschlagen, und wobei ein erstes dem ersten von der Zahnoberfläche (14) reflektierten Licht entsprechendes Bild und ein zweites dem zweiten reflektierten Licht entsprechendes Bild mit der Aufnahmevorrichtung aufnehmbar und erzeugbar sind, wobei die Lichtquellen sequentiell aktivierbar sind.

2. Lichtanalysevorrichtung nach Anspruch 1,
**dadurch gekennzeichnet, dass** die erste und die zweite Lichtquelle (12 und 16) jeweils in unterschiedlichen Winkeln zu der Zahnoberfläche angeordnet sind und diese schräg bestrahlen.

3. Lichtanalysevorrichtung nach einem der Ansprüche 1 oder 2,
**dadurch gekennzeichnet, dass** die erste Lichtquelle (12) und die zweite Lichtquelle (16) mit einer zeitlichen Verzögerung von etwa 2 Sekunden, vorzugsweise von etwa 1/2 Sekunde voneinander einschaltbar sind, um getrennte Bilder zu erzeugen, die dem Licht eines ersten bzw. eines zweiten Strahlenpfads entsprechen.

4. Lichtanalysevorrichtung nach einem der vorhergehenden Ansprüche,
**dadurch gekennzeichnet, dass** sie einen Zeitschalter aufweist, der mit den Lichtquellen (12, 16) und dem Lichtsammelelement (18) verbunden ist und mit dem die erste (12) und zweite (16) Lichtquelle wechselweise zur Erzeugung des ersten und des zweiten Bildes nacheinander aktivierbar ist.

5. Lichtanalysevorrichtung nach Anspruch 1,
**dadurch gekennzeichnet, dass** die zweite Lichtquelle (16) ein zweites Polarisationsfilter (26) zum Polarisieren der zweiten Lichtquelle (16) entlang der ersten Achse aufweist.

6. Lichtanalysevorrichtung nach einem der vorhergehenden Ansprüche,
**dadurch gekennzeichnet, dass** ein zweites Polarisationsfilter (26) zum Polarisieren des Lichts in der ersten Polarisationsachse vorgesehen ist, welches entlang dem Strahlenpfad zwischen der zweiten Lichtquelle (16) und der Zahnoberfläche (14) angeordnet ist, wobei der Strahlenpfad der ersten Lichtquelle (12) nicht durch das zweite Polymerisationsfilter (26) hindurchführt.

7. Lichtanalysevorrichtung nach Anspruch 1,
**dadurch gekennzeichnet, dass** das erste Polarisationsfilter (30) zwischen der Zahnoberfläche (14) und dem Lichtsammelelement (18) entlang des zweiten Strahlenpfads angeordnet ist.

8. Lichtanalysevorrichtung nach einem der Ansprüche 6 oder 7,
**dadurch gekennzeichnet, dass** das erste Polarisationsfilter (30) auch in dem ersten Strahlenpfad angeordnet ist.

9. Lichtanalysevorrichtung nach einem der vorhergehenden Ansprüche,
**dadurch gekennzeichnet, dass** zwischen der Zahnoberfläche (14) und dem Lichtsammelelement (18) eine Sammellinse (40) vorgesehen ist, zur Fokussierung des Bildes der Zahnoberfläche auf das Lichtsammelelement (18).

10. Lichtanalysevorrichtung nach einem der vorhergehenden Ansprüche,
**dadurch gekennzeichnet, dass** die Lichtquellen (12, 16), das Polarisationsfilter (30) und das Lichtsammelelement (18) an einem mit den Händen, insbesondere mit einer Hand bedienbaren Kameragehäuse befestigt sind.

11. Dentalkamera mit einer Lichtanalysevorrichtung nach einem der vorhergehenden Ansprüche, wobei die Dentalkamera Folgendes aufweist:
- ein Kameragehäuse (52), mit welchem die Lichtanalysevorrichtung verbunden ist;
- einen Lichtkanal, der mit der Lichtanalysevorrichtung verbunden ist und der das Licht der ersten (12) und zweiten (16) Lichtquelle durchlässt und Licht, insbesondere radial in den Lichtkanal eintretendes, blockiert,
**dadurch gekennzeichnet, dass** die erste (12) und die zweite (16) Lichtquelle neben der Lichtsammelvorrichtung (18) angeordnet sind, wobei das Lichtsammelelement (18) an dem Kameragehäuse (52) befestigt ist.

12. Dentalkamera nach Anspruch 11,
**dadurch gekennzeichnet, dass** die erste (12) und die zweite (16) Lichtquelle entlang einer Richtung angeordnet sind, die nach vorne zur Position der Zahnoberfläche (14) hin geneigt ist.

13. Dentalkamera nach einem der Ansprüche 11 oder 12,
**dadurch gekennzeichnet, dass**
- die erste (12) und zweite (16) Lichtquelle an dem Kameragehäuse (52) befestigt und separat betreibbar sind, wobei der erste und zweite Pfad in ungleichen Winkeln zu der Position der Zahnoberfläche angeordnet sind;
- das Lichtsammelelement (18) an dem Kameragehäuse befestigt ist.

14. Verfahren für das Abbilden einer Zahnoberfläche (14), das Folgendes aufweist:
- Beaufschlagen der Zahnoberfläche (14) mit einem ersten nicht polarisierten Licht aus einer ersten Lichtquelle (12) und symmetrisch dazu schräg vor der Zahnoberfläche (14), jedoch auf der anderen Seite der Zahnoberfläche (14) angeordneten dritten Lichtquelle (22);
- Sammeln des ersten von der Zahnoberfläche (14) reflektierten Lichts in einem Lichtelement (18);
- insbesondere elektronisches Erzeugen eines ersten Bildes, das dem ersten gesammelten Licht entspricht;
- Beaufschlagen der Zahnoberfläche (14) mit einem zweiten Licht aus einer zweiten Lichtquelle (16) und einer symmetrisch dazu schräg vor der Zahnoberfläche (14), jedoch auf der anderen Seite der Zahnoberfläche (14) angeordneten vierten Lichtquelle . (20), wobei das Licht entlang einer ersten Achse polarisiert wird und wobei die erste und dritte Lichtquelle (12, 22) einerseits und die zweite und vierte Lichtquelle (16, 20) andererseits in verschiedenen Winkeln zu der Zahnoberfläche angeordnet sind und diese schräg bestrahlen;
- Leiten des zweiten von der Zahnoberfläche (14) reflektierten Lichts durch ein Polarisationsfilter (30), um das zweite Licht entlang einer zweiten Achse zu polarisieren;
- Sammeln des zweiten Lichts aus dem Polarisationsfilter (30) in dem Lichtelement (18); und
- insbesondere elektronisches Erzeugen eines zweiten Bildes, das dem zweiten gesammelten Licht entspricht,
- wobei die erste und dritte Lichtquelle (12, 22) und die zweite und vierte Lichtquelle (16, 20) sequentiell aktiviert werden.

15. Verfahren nach Anspruch 14,
**dadurch gekennzeichnet, dass** entweder das erste oder das zweite Licht abgegeben und gesammelt wird, bevor das andere abgegeben und gesammelt wird.

16. Verfahren nach Anspruch 14 oder 15,
**dadurch gekennzeichnet, dass** das erste und das zweite Licht innerhalb eines 2 Sekunden-Abstandes abgegeben werden.

## Claims

1. A light analyzing device to be used in the dental field, comprising:
a first light source (12) that impinges upon a tooth surface (14) with unpolarized light, thus generating first reflected light;
a second light source (16) that impinges upon the tooth surface (14) with a light that is polarized along a first axis, thus generating second reflected light;
a light collecting element (18) that receives the first and the second reflected light from the tooth surface and that comprises a sensing device by means of which an image may be sensed and generated corresponding to the reflected light, wherein the light collecting element (18) comprises a polarizing filter (30) that is arranged so as to polarize the second reflected light along a second axis at an angle to the first axis,
**characterized in that** the light sources (12, 16) are arranged at different angles with respect to the tooth surface and irradiate the surface in an inclined manner, and that a third light source (22) is arranged symmetrically to the first light source (12) in an inclined or slanted position in front of the tooth surface (14), but on the other side of the tooth surface (14), wherein said first and said third light source (22) impinge upon the tooth surface with unpolarized light, and wherein a fourth light source (20) is provided symmetrically to the second light source (16) obliquely in front of the tooth surface (14), but on the other side of the tooth surface (14), wherein said second and said fourth light source (16, 20) impinge upon the tooth surface (14) with polarized light, and wherein a first image that corresponds to the first light reflected by the tooth surface (14), and a second image that corresponds to the second light reflected by the tooth surface, may be sensed and generated with the aid of the image sensing device, wherein the light sources may be activated sequentially.

2. The light analyzing device as claimed in claim 1,
**characterized in that** the first and the second light source (12 and 16) are each arranged at different angles relative to the tooth surface and obliquely irradiate the sur-face.

3. The light analyzing device as claimed in claim 1 or claim 2,
**characterized in that** the first light source (12) and the second light source (16) may be switched on with a time lag of approximately 2 seconds, preferably of approximately 1/2 second, relative to one another, in order to generate separate images that correspond to the light of a first or second radiation or beam path, respectively.

4. The light analyzing device as claimed in one of the preceding claims,
**characterized in that** it comprises a timer that is connected to the light sources (12, 16) and the light collecting element (18), and with the aid of which the first (12) and the second (16) light source may be alternately activated one after the other for generating the first and the second image.

5. The light analyzing device as claimed in claim 1,
**characterized in that** the second light source (16) comprises a second polarizing filter (26) for polarizing the second light source (16) along the first axis.

6. The light analyzing device as claimed in one of the preceding claims,
**characterized in that** a second polarizing filter (26) is provided for polarizing the light along the first polarizing axis, said light being arranged along the beam or radiation path between the second light source (16) and the tooth surface (14), wherein the beam or radiation path of the first light source (12) does not pass through the second polarizing filter (26).

7. The light analyzing device as claimed in claim 1,
**characterized in that** the first polarizing filter (30) is arranged between the tooth surface (14) and the light collecting element (18) along the second beam or radiation path.

8. The light analyzing device as claimed in one of the claims 6 or 7,
**characterized in that** the first polarizing filter (30) is also arranged along the first beam or radiation path.

9. The light analyzing device as claimed in one of the preceding claims,
**characterized in that** a collecting lens (40) is provided between the tooth surface (14) and the light collecting element (18) for focussing the image of the tooth surface on the light collecting element (18).

10. The light analyzing device as claimed in one of the preceding claims,
**characterized in that** the light sources (12, 16), the polarizing filter (30) and the light collecting element (18) are attached to a camera housing that may be operated with both hands or particularly with one hand.

11. A dental camera having a light analyzing device as claimed in one of the preceding claims, said dental camera comprising:
a camera housing (52) associated with the light analyzing device;
a light channel connected with the light analyzing device and adapted to allow the light from the first (12) and the second (16) light source to pass therethrough, and to block light from entering the light channel, in particular light that enters the light channel radially,
**characterized in that** the first (12) and the second (16) light source are arranged adjacent to the light collecting device (18) that is attached to the camera housing (52).

12. The dental camera as claimed in claim 11,
**characterized in that** the first (12) and the second (16) light source are arranged along a direction that is inclined forward towards the position of the tooth surface (14).

13. The dental camera as claimed in one of the claims 11 or 12,
**characterized in that**
- the first (12) and the second (16) light source are attached to the camera housing (52) and may be operated separately, wherein the first and the second path are arranged at different angles with respect to the position of the tooth surface;
- the light collecting element (18) is attached to the camera housing.

14. A method for imaging a tooth surface (14), said method comprising the following steps:
- impinging upon the tooth surface (14) with a first unpolarized light from a first light source (12) and further from a third light source (22) that is arranged symmetrically to the first light source (12) obliquely in front of the tooth surface (14), but on the other side of the tooth surface (14);
- collecting the first light reflected by the tooth surface (14) in a light element (18);
- in particular electronically generating a first image that corresponds to the first collected light;
- impinging upon the tooth surface (14) with a second light from a second light source (16) and from a fourth light source (20) that is symmetrically arranged thereto obliquely in front of the tooth surface (14), but on the other side of the tooth surface (14), wherein the light is polarized along a first axis and wherein the first and the third light source (12, 22) on the one hand, and the second and fourth light source (16, 20) on the other hand, are arranged at different angles with respect to the tooth surface and obliquely irradiate
thereon;
directing the second light reflected by the tooth surface (14) through a polarizing filter (30) for polarizing the second light along a second axis;
collecting the second light from the polarizing filter (30) in the light element (18); and
in particular electronically generating a second image that corresponds to the second collected light;
wherein the first and the third light source (12, 22) as well as the second and the fourth light source (16, 20) are activated sequentially.

15. The method as claimed in claim 14,
**characterized in that** either the first or the second light is emitted and collected before the other one is emitted and collected.

16. The method as claimed in claim 14 or 15,
**characterized in that** the first and the second light are emitted within 2 seconds from one another.

## Revendications

1. Dispositif d'analyse de lumière pour le domaine dentaire qui présente:
une première source lumineuse (12) qui frappe une surface dentaire (14) avec de la lumière non-polarisée, où une première lumière réfléchie est produite;
une deuxième source lumineuse (16) qui frappe la surface dentaire (14) avec de la lumière polarisée le long d'un premier axe, où une deuxième lumière réfléchie est produite ; un élément de collecte de la lumière (18) qui recueille la première et la deuxième lumière réfléchie de la surface dentaire (14) et présente un dispositif d'enregistrement, avec lequel une image correspondant à la lumière réfléchie peut être enregistrée et produite, où l'élément de collecte de la lumière (18) présente un filtre de polarisation (30) qui est disposé de telle sorte qu'il polarise la deuxième lumière réfléchie le long d'un deuxième axe à un angle au premier axe,
**caractérisé en ce que** les sources lumineuses (12, 16) sont disposées à différents angles à la surface dentaire et illuminent celle-ci en diagonale et que symétriquement à la première source lumineuse (12) en diagonale devant la surface dentaire (14) toutefois de l'autre côté de la surface dentaire (14) une troisième source lumineuse (22) est disposée, où la première et la troisième source lumineuse (20) frappent la surface dentaire avec de la lumière non-polarisée, et où symétriquement à la deuxième source lumineuse (16) en diagonale devant la surface dentaire (14), toutefois de l'autre côté de la surface dentaire (14), une quatrième source lumineuse (20) est prévue, où la deuxième et la quatrième source lumineuses (16, 20) frappent la surface dentaire (14) avec de la lumière polarisée, et où une première image correspondant à la première lumière réfléchie de la surface dentaire (14) et une deuxième image correspondant à la deuxième lumière réfléchie peuvent être enregistrées et produites avec le dispositif d'enregistrement, où les sources lumineuses peuvent être activées en séquence.

2. Dispositif d'analyse de lumière selon la revendication 1,
**caractérisé en ce que** la première et la deuxième source lumineuse (12 et 16) sont disposées respectivement à des angles différents à la surface dentaire et illuminent celle-ci en diagonale.

3. Dispositif d'analyse de lumière selon la revendication 1 ou 2,
**caractérisé en ce que** la première source lumineuse (12) et la deuxième source lumineuse (16) peuvent être activées avec un retard temporel l'un par rapport à l'autre d'environ 2 secondes, de préférence environ 1/2 seconde, pour produire les images distinctes qui correspondent à la lumière d'un premier ou d'un deuxième trajectoire de rayon.

4. Dispositif d'analyse de lumière selon l'une des revendications précédentes,
**caractérisé en ce qu'**elle présente un interrupteur à minuterie qui est connectée à des sources lumineuses (12 et 16) et à l'élément de collecte de la lumière (18) et avec laquelle la première (12) et la deuxième (16) sources lumineuses sont alternativement activées pour produire la première et la deuxième image.

5. Dispositif d'analyse de lumière selon la revendication 1,
**caractérisé en ce que** la deuxième source lumineuse (16) présente un deuxième filtre de polarisation (26) pour polariser la deuxième source lumineuse (16) le long du premier axe.

6. Dispositif d'analyse de lumière selon l'une des revendications précédentes,
**caractérisé en ce qu'**un deuxième filtre de polarisation (26) est prévu pour polariser la lumière dans le premier axe de polarisation qui est disposée le long du trajectoire de rayon entre la deuxième source lumineuse, {16} et la surface dentaire (14), où la trajectoire de rayon de la première source lumineuse (12) ne traverse pas le deuxième filtre de polymérisation (26).

7. Dispositif d'analyse de lumière selon la revendication 1,
**caractérisé en ce que** le premier filtre de polarisation (30) est disposé entre la surface dentaire (14) et l'élément de collecte de la lumière (18) le long du deuxième trajectoire de rayon.

8. Dispositif d'analyse de lumière selon l'une des revendications 6 ou 7,
**caractérisé en ce que** le premier filtre de polarisation (30) est disposé aussi dans la première trajectoire de rayon.

9. Dispositif d'analyse de lumière selon l'une des revendications précédentes,
**caractérisé en ce qu'**entre la surface dentaire (14) et l'élément de collecte de la lumière (18), une lentille de convergence (40) est prévue, pour focaliser l'image de la surface dentaire sur l'élément de collecte de la lumière (18).

10. Dispositif d'analyse de lumière selon l'une des revendications précédentes,
**caractérisé en ce que** les sources lumineuses (12, 16), le filtre de polarisation (30) et l'élément de collecte de la lumière (18) sont attachés à un boîtier de caméra contrôlable avec les mains en particulier avec une main.

11. Caméra dentaire avec un dispositif d'analyse de lumière selon l'une des revendications précédentes, où la caméra dentaire présente:
- un boîtier de caméra auquel le dispositif d'analyse de lumière est connecté;
- un canal de lumière qui est connecté au dispositif d'analyse de lumière et qui laisse passer la lumière de la première (12) et la deuxième source lumineuse et bloque, en particulier la lumière qui entre radialement dans le canal de lumière, **caractérisé en ce que** la première et la deuxième (16) source lumineuse sont disposées à côté du dispositif collectif de lumière (18), où l'élément de collecte de la lumière (18) est attaché au boîtier de la caméra.

12. Caméra dentaire selon la revendication 11,
**caractérisée en ce que** la première et la deuxième (16) sources lumineuses sont disposées le long d'une direction qui est inclinée vers l'avant relative à la position.

13. Caméra dentaire selon l'une des revendications 11 ou 12, **caractérisée en ce que**
- la première et la deuxième (16) source lumineuses sont attachées au boîtier de la caméra (52) et peuvent être opérées séparément, où le premier et la deuxième trajectoire sont disposés à des angles inégaux par rapport à la position de la surface dentaire;
- l'élément de collecte de la lumière (18) est attaché au boîtier de la caméra.

14. Procédure pour faire une représentation d'une surface dentaire (14) qui présente les caractéristiques suivantes:
- frapper la surface dentaire (14) avec une première lumière non-polarisée dΔune première source lumineuse (12) et d'une troisième source lumineuse (22) disposée symétriquement en diagonale devant la surface dentaire (14) toutefois de l'autre côté de la surface dentaire (14) et;
- recueillir la première lumière réfléchie de la surface dentaire (14) dans un élément lumineux (18);
- en particulier produire électroniquement une première image qui correspond à la première lumière collectée;
- frapper la surface dentaire (14) avec une deuxième lumière d'une deuxième source lumineuse (16) et une quatrième source lumineuse (20) disposée symétriquement à celle-ci en diagonale devant la surface dentaire (14), toutefois de l'autre côté de la surface dentaire (14), où la lumière est polarisée le long d'un premier axe et où d'une part la première et troisième sources lumineuses (12, 22) et d'autre part la deuxième et la quatrième sources lumineuses (16, 20), sont disposés à différents angles par rapport à la surface dentaire et illuminent celle-ci en diagonale;
- guider la deuxième lumière réfléchie de la surface dentaire (14) à travers un filtre de polarisation, afin de polariser la deuxième lumière le long d'un deuxième axe; - recueillir la deuxième lumière d'un filtre de polarisation (30) dans l'élément de lumière (18); et
- en particulier produire électroniquement une deuxième image qui correspond à la deuxième lumière recueillie, où les première et troisième sources lumineuses (12, 22) et les deuxième et quatrième sources lumineuses (16, 20) sont activées en séquence.

15. Procédure selon la revendication 14,
**caractérisée en ce que** soit la première, soit la deuxième lumière est émise et recueillie, avant que l'autre ne soit émise et collectée.

16. Procédure selon la revendication 14 ou 15,
**caractérisée en ce que** la première et la deuxième lumière sont émises avec un écart de 2 seconds.
